(19) Europäisches Patentamt
European Patent Office
Office européen des brevets

(11) EP 0 849 392 B1

(12) **EUROPEAN PATENT SPECIFICATION**

(45) Date of publication and mention of the grant of the patent: **17.04.2002 Bulletin 2002/16**

(51) Int Cl.[7]: **D06M 15/356**, D06M 15/576, C08F 16/28, C07C 271/24, C07C 271/28

(21) Application number: **97122276.5**

(22) Date of filing: **17.12.1997**

(54) **Fluoroalkylated allylurethane, its copolymer and water- and oil-repellent, anti-soiling processing agent containing the copolymer as an effective component**

Fluoroalkylierte Allylurethane, daraus hergestellte Copolymere, und wasser-, öl- und fleckabweisendes Behandlungsmittel enthaltend das Copolymer als wirksamen Bestandteil

Uréthanes allyliques fluoroalcoyleés, leurs copolyméres et agent de traitement hydrofuge, oléophobe et antitâches contenant le copolymère comme composé actif

(84) Designated Contracting States:
**BE DE**

(30) Priority: **20.12.1996 JP 35482496**

(43) Date of publication of application:
**24.06.1998 Bulletin 1998/26**

(73) Proprietor: **NIPPON MEKTRON, LTD.**
**Minato-ku Tokyo (JP)**

(72) Inventors:
- **Yamamoto, Kazuhiro**
**Kitaibaraki City, Ibaraki (JP)**
- **Ishida, Yuzuru**
**Kitaibaraki City, Ibaraki (JP)**

(74) Representative:
**Hansen, Bernd, Dr. Dipl.-Chem. et al**
**Hoffmann Eitle,**
**Patent- und Rechtsanwälte,**
**Arabellastrasse 4**
**81925 München (DE)**

(56) References cited:
**EP-A- 0 103 752**
**EP-A- 0 225 826**
**EP-A- 0 414 155**
**GB-A- 1 102 903**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

**Description**

1. FIELD OF THE INVENTION

The present invention relates to a novel fluoroalkylated allylurethane, its copolymer and a water- and oil-repellent, anti-soiling processing agent containing the copolymer as an effective component, and more particularly to a copolymer capable of forming a water- and oil- repellent, which can give a distinguished water- and oil- repellency and a distinguished anti-dry soiling property for fibers, particularly carpets and a novel fluoroalkylated allylurethane as a comonomer component for the copolymer.

2. RELATED ART

Various fluorinated polymers have been so far known as water- and oil- repellents for fibers. For example, JP-B-47-6600 (corresponding to DE 1 795 261) discloses that bulk homopolymerization products of yellowish viscous oil obtained by reaction of tolylenediisocyanate (a mixture consisting of about 80% of 2,4-isomer and about 20% 2,6-isomer) with fluoroalcohol ($C_{17}F_{35}CH_2OH$), followed by reaction with allyl alcohol show a distinguished water- and oil-repellency for natural fibers. However, such viscous oil-like monomers undergo solution polymerization because of its good solubility in an organic solvent, but their emulsification is too hard and thus no aqueous emulsion can be obtained in case of the emulsion polymerization, resulting in formation of precipitates during the polymerization.

JP-B-3-4542 (corresponding to EP 225 826) discloses that (co)polymers for the protection of leathers can be obtained by solution polymerization from fluorinated acryl monomers prepared by reaction of 2,4-tolylenediisocyanate with fluoroalcohol ($C_6F_{13}CH_2CH_2OH$), followed by reaction with 2-hydroxyethyl methacrylate, but the fluorinated acryl monomers are poor in emulisifability and thus their emulsion polymerization is also hard to conduct.

JP-A-3-76873 (corresponding to EP 414 155) discloses an anti-soiling processing agent obtained by emulsion polymerization between fluorourethane acrylate, prepared by reaction of triisocyanate (e.g. hexamethylenediisocyanate trisbiuret) with fluoroalcohol (e.g. $C_9F_{19}CH_2CH_2OH$), followed by reaction with hydroxylated methacrylate ester [e.g. $CH_2{=}C(CH_3)COO(CH_2CH_2O)nH$], and methyl methacrylate or the like. However, pretreatments must be carried out before the emulsion polymerization, i.e. first emulsification by a high-speed, rotating homomixer and second emulsification by a high pressure homogenizer. Thereafter, the emulsion polymerization reaction is to be carried out. As mentioned above, fluorourethane has problems of difficult emulsification and formation of precipitates during the emulsion polymerization based on the ordinary emulsification process, and thus there is an additional problem of necessity for use of an expensive emulsification apparatus as an emulsification means. GB 1,102,903 provides fluorine-containing compounds having a group of the formula

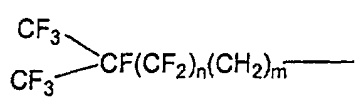

n being an integer from 2 to 20 inclusive and m being 0 or an integer from 1 to 20 inclusive, bonded to one end position. The compounds are used in water-repellent and oil-repellent compositions.

EP-A-0 103 752 discloses a stainproofing agent containing as an active ingredient a polyfluoroalkyl group-containing compound represented by the following general formula and having a molecular weight of from 800 to 3000:

$$(R_1\text{-}X\text{-}A\text{-}CONH)_a\text{—}W\text{—}(NHCO\text{-}A'\text{-}Z)_{3-a}$$

where $R_1$ is a polyfluoroalkyl group having from 1 to 20 carbon atoms, X is -R-, -CON($R^1$)-Q- or -$SO_2$N($R^1$)-Q- (where R is a divalent alkylene group, $R^1$ is a hydrogen atom or a lower alkyl group and Q is a divalent organic group), each of A and A' is -O-, -S- or -N(Z')- (where Z' is a hydrogen atom or a monovalent organic group), Z is a monovalent organic group, W is a trivalent organic group, a is an integer of 1, 2 or 3.

SUMMARY OF THE INVENTION

An object of the present invention is to provide a copolymer capable of forming a water- and oil- repellent, anti-soiling processing agent; which can give a distinguished water-and oil- repellency and a distinguished anti-dry

soiling property for fibers, particularly carpets, etc., which can be obtained in an aqueous emulsion by the ordinary emulsion polymerization method and also to provide a novel fluoroalkylated allylurethane as a comonomer component for the copolymer.

According to the present invention, there is provided a novel fluoroalkylated allylurethane represented by the following general formula:

$$RfROCONHR'\ NHCOOC_n\ H_{2n}OCH_2CH{=}CH_2$$

where Rf is a perfluoroalkyl group having 2 to 20 carbon atoms; R is a divalent organic group; R' is a diisocyanate residue; and n is an integer of 2 to 6.

The present novel fluoroalkylated allylurethane can give an aqueous emulsion of copolymer useful as an effective water - and oil- repellent, anti-soiling processing agent by emulsion polymerization with at least one of a methacrylate ester, acrylate ester and vinylidene chloride.

DETAILED DESCRIPTION OF THE INVENTION

The present novel fluoroalkylated allylurethane represented by the above general formula can be prepared by (1) reaction of a diisocyanate compound having two NCO groups of different reactivity in the molecule with a fluoroalcohol represented by the following general formula RfROH, where Rf and R have the same meanings as defined above, followed by reaction with alkyleneglycol monoallyl ether or by (2) reaction of a diisocyanate having two NCO groups of different reactivity in the molecule with alkyleneglycol monoallyl ether, followed by reaction with fluoroalcohol. In these individual reactions, the diisocyanate may offer different sites of substitution with the counterpart reactants, depending upon the sequence of adding equimolar amounts of the counterpart reactants to the diisocyanate, but the difference in the site of substitution on the diisocyanate gives no significant difference to properties of water- and oil-repellent, anti-soiling processing agent containing copolymers of the present fluoroalkylated allylurethane.

Diisocyanate compounds each having two NCO groups of different reactivity in the molecule such as aromatic diisocyates (e.g. 2,4-tolylenediisocyanate, etc.) and alicyclic diisocyates (e.g. 3-isocyanatomethyl-3,5,5-trimethylcyclohexylisocyanate, etc.) can be used in the present invention as the diisocyanate compound. When a diisocyanate compound having NCO groups of same reactivity is used, there is a high possibility of formation of difluorourethane or diallylurethane, and the desired fluoroalkylated allylurethane will be hard to obtain.

Fluoroalcohols whose Rf group is a straight or branched perfluoroalkyl group having 2 to 20 carbon atoms and whose R group is a straight or branched alkylene group of $C_mH_{2m}$, where m is an integer of 2 to 6, a $SO_2NR_1CH_2CH_2$ group, where $R_1$ is a lower alkyl group having 1 to 5 carbon atoms, a $CON(C_2H_5)CH_2CH_2$ group, a $CON(CH_3)CH_2CH_2$ group, a $CH_2CH(OH)CH_2$ group, etc. can be used in the present invention as the fluoroalcohol. Preferably, a fluoroalcohol whose Rf group is a straight perfluoroalkyl group having 6 to 16 carbon atoms and whose R group is a $CH_2CH_2$ group is used in the present invention.

Alkyleneglycol monoallyl ether for use in the present invention includes, for example, ethyleneglycol monoallyl ether, propyleneglycol monoallyl ether, butyleneglycol monoallyl ether, etc. Preferably, ethyleneglycol monoallyl ether is used in the present invention from the viewpoint of anti-dry soiling property.

Reaction of the diisocyanate compound, the fluoroalcohol and the alkyleneglycol monoallyl ether can be carried out in an inert organic solvent or a polymerizable monomer. The inert organic solvent includes, for example, ketones such as acetone, methyl ethyl ketone, methyl isobutyl ketone, etc. esters such as ethyl acetate, butyl acetate, methyl propionate, methyl acetate,etc. and aromatic hydrocarbons such as toluene, etc.

As the polymerizable monomer, those having no active hydrogen can be used. Preferably, such comonomer counterpart of fluoroalkylated allylurethane as a reaction product of the diisocyanate compound, the fluoroalcohol and the alkyleneglycol monoallyl ether as methacrylate esters, acrylate esters or vinylidene chloride is used. The methacrylate ester for use in the present invention includes, for example, methyl methacrylate, ethyl methacrylate, n-butyl methacrylate, isobutyl methacrylate, t-butyl methacrylate, 2-ethylhexyl methacrylate, benzyl methacrylate, cyclohexyl methacrylate, lauryl methacrylate, stearyl methacrylate, etc. The acrylate esters for use in the present invention are acrylates corresponding to the above-mentioned methacrylates.

When the amount of the solvent, is too small, it will be difficult to control heat generation due to the exothermic reaction, whereas too a large amount of the solvent makes the reaction inefficient. From these viewpoints, about 0.5 to about 10 parts by weight, preferably about 0.5 to about 2 parts by weight of the solvent is used per one part by weight of the fluoroalcohol.

A catalyst is used in the reaction. The calalyst includes, for example, an organotin compound such as dibutyl tin dilaurate, dibutyl tin maleate, dimethyldichloro tin, etc., a tertiary amine such as triethylamine, tripropylamine, tributylamine, etc., manganese, cobalt, zirconium, lead, barium nitrate, etc. Preferably, dibutyl tin maleate is used from

the viewpoint of reaction rate, etc.

The reaction using the catalyst is carried out at room temperature or higher, preferably about 40° to about 100°C.

The above-mentioned reaction (1) can be carried out in the following manner:

A diisocyanate compound, a catalyst and a solvent are charged into a reactor, and then a fluoroalcohol solution in a solvent is dropwise added thereto, or a diisocyanate compound and a catalyst are charged into a reactor and then a fluoroalcohol solution in a solvent is dropwise added thereto. When the Rf group of fluoroalcohol has 8 or more carbon atoms, the fluoroalcohol is in a solid state and thus dropwise addition as a solution in the solvent is carried out, whereas when the Rf group has 6 or less carbon atoms, the fluoroalcohol is in a liquid state and can be directly dropwise added thereto without its dissolution in the solvent. Rate of dropwise addition depends upon a reaction scale, a reactor size, etc., but too a high rate of dropwise addition may cause runaway of reaction because of the exothermic reaction. Thus, it is desirable to conduct dropwise addition slowly, while monitoring the reaction temperature.

After the dropwise addition of fluoroalcohol, aging is carried out for about 0.1 to about 1 hour, and a vibration absorption of NCO can be observed at 2270 $cm^{-1}$ by infrared spectroscopic analysis of the reaction solution.

Thereafter, alkyleneglycol monoallyl ether is dropwise added thereto to completely consume NCO. When the urethanization reaction is completed, the absorption at 2270 $cm^{-1}$ disappears completely. To prevent polymerization reaction of the product, it is preferable to conduct dropwise addition of alkylglycol monoallyl ether by adding about 300 to about 1,000 ppm of a polymerization inhibitor such as hydroquinone, etc. (on the basis of fluoroalcohol) to the alkylglycol monoallyl ether through its dissolution into the allkylglycol monoallyl ether.

The above-mentioned reaction (2) can be carried out substantially in the same manner as the reaction (1). However, the reaction product of reaction (2) is different from that of reaction (1), as mentioned above. For example, in case of reaction of 2,4-tolylenediisocyanate with fluoroalcohol and alkyleneglycol monoallyl ether, the fluoroalcohol undergoes addition and substitution at the 4-position in the reaction (1), but on the 2-position in the reaction (2), and the alkyleneglycol monoallyl ether undergoes addition and substitution at the 2-position in the reaction (1), but at the 4-position in the reaction (2).

The fluoroalkylated allylurethane as a product of these reactions can be copolymerized with at least one of a methacrylate ester, a acrylate ester and vinylidene chloride as mentioned above. These polymerizable monomer as comonomer may be added to the fluoroalkylated allylurethane, for the first time, at the stage of polymerization reaction or the polymerizable monomer used as a solvent for preparation of the fluoroalkylated allylurethane can be used as such.

In the copolymerization reaction, an acrylic monomer having a cross-linkable group such as acrylamide, N-methylolacrylamide, N-(methoxymethyl)acrylamide, N-(butoxymethyl)acrylamide, glycidyl acrylate, glycidyl methacrylate, etc. can be also copolymerized.

About 30 to about 90% by weight, preferably about 45 to about 80% by weight, of the fluoroalkylated allylurethane can be used on the basis of sum total of comonomers. Since the copolymerization proceeds substantially quantitatively, the charging proportions of the comonomers perse correspond to copolymerization proportion. From the viewpoint that hard copolymers are preferable to soft copolymers to give an anti-dry soiling property, in other words, copolymers having a higher Tg than room temperature are preferable, it is desirable to use at least two polymerizable monomers rather than a single polymerizable monomer. The acrylic monomer having a cross-linkable group can be used in a proportion of not more than about 10% by weight, preferably about 0.1 to about 5% by weight on the basis of sum total of comonomers.

It is preferable from the viewpoint of use of the copolymers, (i.e. water- and oil-repellent, anti-soiling processing agent) to conduct copolymerization reaction by emulsion polymerization. Any of nonionic, cationic, anionic and amphoteric surfactants can be used as an emulsifying agent for the emulsion polymerization. Specifically, the emulsifying agent includes nonionic surfactants such as polyoxyethylene lauryl ether, polyoxyethylene cetyl ether, polyoxyethylene oleyl ether, polyoxyethylene octylphenyl ether, polyoxyethylene nonylphenyl ether, sorbitan fatty acid ester, polyoxyethylene sorbitan fatty acid ester, etc., cationic surfactants such as quaternary ammonium salts, etc., anionic surfactants such as sodium laurylsulfate, ammonium laurylsulfate, sodium dodecylbenzenesulfonate, etc., and amphoteric surfactants such as lauryl betaine, stearyl betaine, etc. From the viewpoint of stability of simultaneous use of other chemicals for fiber treatment when the copolymers are used as an anti-soiling processing agent, the nonionic surfactants having a good stability with anionic or cationic chemicals are most preferable.

Any polymerization catalyst can be used, so long as it is water-soluble. Preferable are ammonium persulfate, potassium persulfate, 2,2'-azobis(2-amidinopropane) dihydrochloride, etc. When the polymerization catalyst is used together with a reducing agent to form a redox system, the polymerization initiation temperature is lowered. That is, the polymerization can be initiated at about 20°C, generally at about 30°C, but the upper limit temperature is not more than 100°C, because an aqueous medium is used.

An aqueous emulsion resulting from the copolymerization reaction has a solid concentration of about 20 to about 30% by weight. When the aqueous emulsion is used as a water- and oil-repellent, anti-soiling agent, it is diluted

to a solid concentration of about 0.2 to about 2% by weight.

The water- and oil-repellent, anti-soiling processing agent containing the present copolymer as an effective component can be effectively applied to fiber products, particularly carpets by such means as spraying, dipping, foam application, etc., followed by drying at a temperature of about 105° to about 140°C for about 1 to about 30 minutes.

The present invention provides a novel fluoroalkylated allylurethane which can be copolymerized with at least one of methacrylate esters, acrylate esters and vinylidene chloride by emulsion polymerization. Emulsion polymerization can be readily carried out by an ordinary emulsification means. The resulting copolymers can give a distinguished water- and oil-repellency and a distinguished anti-dry soiling property for fiber products such as carpets, etc.

PREFERRED EMBODIMENTS OF THE INVENTION

The present invention will be described in detail below, referring to Examples.

EXAMPLE 1

174g (one mole) of 2,4-tolylenedusocyanate, 1.6g of dibutyl tin dilaurate and 666g of MIBK (methyl isobutyl ketone) were charged into a separable glass flask with a stirrer, and the flask inside temperature was started to increase while stirring the mixture solution. When the inside temperature reached 50°C, 504g (one mole) of fluoroalcohol $RfC_2H_4OH$ (average molecular weight: 504; Rf: a perfluoroalkyl group having an average carbon atom number of 8.8) as dissolved in 506g of MIBK was dropwise added to the mixture solution, while monitoring the inside temperature. When the inside temperature was slowly elevated to 70°C due to heat generation, the rate of dropwise addition was adjusted to keep that temperature. Time required from the start to the end of dropwise addition was 3 hours. After the dropwise addition, the reaction solution was subjected to aging for 30 minutes, and then a small amount of the reaction solution was sampled for infrared spectroscopic analysis. Absorption due to the absorption of -NCO was observed at 2270 $cm^{-1}$.

102g (one mole) of ethyleneglycol monoallyl ether dissolving 0.4g of hydroquinone was slowly dropwise added to the reaction solution so as not to cause abrupt heat generation. Rate of dropwise addition was adjusted to keep the reaction temperature at 70°C. Time required for the dropwise addition was 3 hours. After aging for one hour, a small amount of the reaction solution was sampled for infrared spectroscopic analysis. It was found that the absorption at 2270 $cm^{-1}$ due to the absorption of -NCO disappeared. The reaction solution was 1934g of a clear dark brown fluoroalkylated allylurethane solution in MIBK. The reaction yield calculated from the solid concentration (40% by weight) was 99%.

A portion of the MIBK solution was sampled and the solvent was distilled off from the sample. Brownish solid residue was determined for the melting point (no constant melting point is shown due to a mixture of perfluoroalkyl groups having different carbon atom numbers) and also for infrared absorption spectra (IR absorption) and nuclear magnetic resonances. The results are given below:

| | |
|---|---|
| Melting point: | 80-86°C |
| IR absorption: | 1700 $cm^{-1}$ (-NHCOO-) |
| | 1100-1300 $cm^{-1}$ (-CF) |

$^1H$-NMR($CD_3COCD_3$):

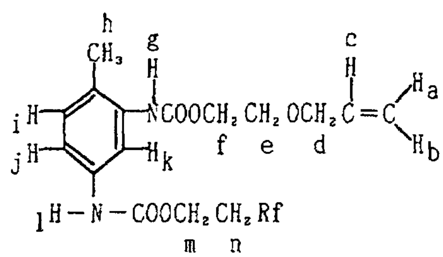

| | Chemical shift (ppm) | Proton number |
|---|---|---|
| a | 5.13 | 1 |
| b | 5.28 | 1 |
| c | 5.89 | 1 |
| d | 4.02 | 2 |
| e | 3.66 | 2 |
| f | 4.24 | 2 |
| g | 7.83 | 1 |
| l | 8.73 | 1 |
| h | 2.25 | 3 |
| i | 7.10 | 1 |
| j | 7.30 | 1 |
| k | 7.82 | 1 |
| m | 4.47 | 2 |
| n | 2.27 | 2 |
| | | (Total 21) |

$^{19}F$-NMR($CD_3COCD_3$):

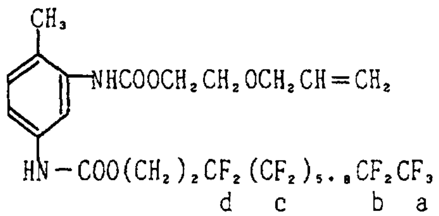

| | Chemical shift($CFCl_3$~) | F number |
|---|---|---|
| a | -80.13 | 3 |
| b | -125.16 | 2 |
| c | -120.58~-122.44 | 11.6 |
| d | -112.35 | 2 |
| | | (Total 18.6) |

EXAMPLES 2 TO 10

A corresponding amount of the fluoroalkylated allylurethane solution in MIBK obtained in Example 1 to the solid weight calculated from the solid concentration was weighed out and charged into a separable glass flask with a stirrer together with a given amount of MMA (methyl methacrylate), $VdCl_2$ (vinylidene chloride) or BzA (benzyl acrylate) as monomer (a) and a given amount of iBMA (isobutyl methacrylate), EMA (ethyl methacrylate) or MMA as monomer (b).

Then, the following compounds were further charged therein:

| | |
|---|---|
| Polyoxyethylene nonylphenyl ether (Emulgen 950, a trademark of nonionic surfactant commercially available from Kao Corp., Japan) | 3.3g |
| n-Dodecyl mercaptan | 0.25g |
| Water (Example 2) | 307.5g |
| " (Example 3) | 295g |
| " (Examples 4 to 10) | 270g |

the mixture was emulsified by a high speed rotating homomixer (Ultra-talax T25, tradename of a mixer commercially available from IKA, Japan) at 13000 rpm for 5 minutes.

Thereafter, the flask was flushed with a nitrogen gas for 30 minutes with stirring. After elevation of the flask inside temperature to 40°C by heating, a given amount of N-MAM (N-methylol acrylamide), M-MAM (N-methoxymethyl acrylamide) or ACM (acrylamide) (not used in Examples 4 and 5), and an aqueous solution containing 2g of 2,2'-azobis (2-amidinopropane) dihydrochloride (V-50, a trademark of a product commercially available from Wako Junyaku K.K., Japan) dissolved in 30g of water were added to the reaction system, followed by polymerization reaction at 70°C for 4 hours. Stable, milky-white, aqueous emulsions each having a solid concentration of 25% by weight were obtained in all the Examples.

| Example No. | Fluoroalkylated allylurethane Solid amount (g) | Copolymerizable monomer (g) | | |
|---|---|---|---|---|
| | | (a) | (b) | (c) |
| 2 | 45 | MMA 30 | i BMA 23 | N-MAM 2 |
| 3 | 50 | MMA 37 | iBMA 11 | N-MAM 2 |
| 4 | 60 | MMA 30 | i BMA 10 | - |
| 5 | 60 | MMA 30 | EMA 10 | - |
| 6 | 60 | MMA 30 | EMA 9 | M-MAM 1 |
| 7 | 60 | MMA 20 | EMA 19 | M-MAM 1 |
| 8 | 60 | MMA 30 | EMA 9 | ACM 1 |
| 9 | 60 | $VdCl_2$ 20 | MMA 19 | ACM 1 |
| 10 | 60 | BzA 30 | EMA 9 | ACM 1 |

EXAMPLE 11

17.4g (0.1 mole) of 2,4-tolylenediisocyanate and 0.16g of dibutyl tin dilaurate were charged into a separable glass flask with a stirrer, and the flask inside temperature was started to increase, while stirring the mixture. When the inside temperature reached to 50°C, 50.4g (0.1 mole) of fluoroalcohol $RfC_2H_4OH$ (average molecular weight: 504; Rf: a perfluoroalkyl group having an average carbon atom number of 8.8) as dissolved in 39g of MMA and 13g of EMA was dropwise added to the mixture solution slowly, while monitoring the inside temperature. When the inside temperature was slowly elevated to 70°C due to the exothermic reaction, the rate of dropwise addition was adjusted so as to keep that temperature. Time required from the start to the end of the dropwise addition was one hour. After the dropwise addition, the reaction solution was subjected to aging for 30 minutes and then a small amount of the reaction solution was sampled and subjected to infrared spectroscopic analysis. Absorption due to -NCO absorption was observed at 2270 $cm^{-1}$.

Then, 10.2g (0.1 mole) of ethyleneglycol monoallyl ether dissolving 0.04g of hydroquinone was slowly dropwise added to the reaction solution so as not to cause vigorous heat generation. Rate of dropwise addition was adjusted to keep the reaction temperature at 70°C. Time required for the dropwise addition was one hour. After further aging for one hour, a small amount of the reaction solution was sampled and subjected to infrared spectroscopic analysis. It was found that the absorption at 2270 $cm^{-1}$ due to the -NCO absorption disappeared. Amount of the reaction solution was 128.9g and the reaction yield was 99%.

Whole amount of the reaction product was charged into a separable glass flask and then 4.3g of polyoxyethylene phenyl ether (Emulgen 950), 0.35g of n-dodecyl mercaptane and 357g of water were added thereto, followed by emulsification by a high speed, rotating homomixer (Ultra-talax T25) at 13000 rpm for 5 minutes.

Thereafter, the flask was flushed with a nitrogen gas for 30 minutes with stirring. After elevation of the inside temperature to 40°C by heating, 1.3g of M-MAM and 2.6g of 2,2'-azobis(2-amidinopropane) dihydrochloride (V-50) as dissolved in 30g of water were added to the reaction system, followed by polymerization reaction at 70°C for 4 hours, whereby a stable, milky-white aqueous emulsion having a solid concentration of 25 % by weight was obtained.

EXAMPLE 12

In Example 11, reaction of 2,4-tolylenediisocyanate with ethyleneglycol monoallyl ether was carried out at first, and then reaction with $RfC_2H_4OH$ was carried out. A small amount of the reaction solution was sampled, and the brownish solid residue from removal of the solvent tehrefrom by distillation was determined for melting point, IR absorption and nuclear magnetic resonances. The results are as follows:

Melting point: 71-89°C

IR absorption: 1700 $cm^{-1}$ (-NHCOO-) 1100-1300 $cm^{-1}$ (-CF)

$^1H$-NMR($CD_3COCD_3$) :

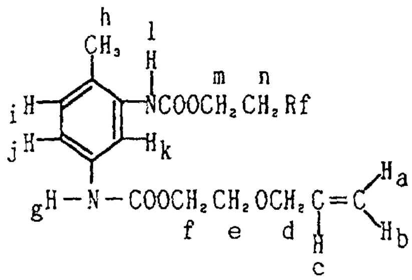

| | Chemical shift(ppm) | Proton number |
|---|---|---|
| a | 5.13 | 1 |
| b | 5.28 | 1 |
| c | 5.90 | 1 |
| d | 3.99 | 2 |
| e | 3.65 | 2 |
| f | 4.24 | 2 |
| g | 8.61 | 1 |
| l | 7.99 | 1 |
| h | 2.23 | 3 |
| i | 7.11 | 1 |
| j | 7.32 | 1 |
| k | 7.80 | 1 |
| m | 4.47 | 2 |
| n | 2.70 | 2 |
| | | (Total 21) |

$^{19}F$-NMR($CD_3COCD_3$):

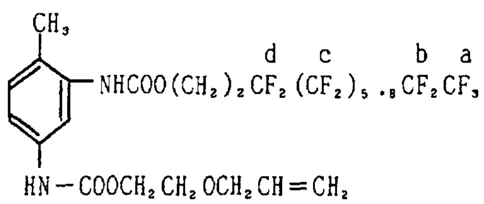

| | Chemical shift($CFCl_3$~) | F number |
|---|---|---|
| a | -80.19 | 3 |
| b | -125.22 | 2 |
| c | -120.77~-122.51 | 11.6 |
| d | -112.38 | 2 |
| | | (Total 18.6) |

Then, the reaction product was subjected to emulsion polymerization under the same conditions as in Example 11, whereby a stable, milky-white aqueous emulsion having a solid concentration of 25% by weight was obtained.

EXAMPLE 13

22.2g (0.1 mole) of 3-isocyanatomethyl-3,5,5-trimethylcyclohexyl isocyanate and 0.17g of dibutyl tin laurate were charged into a separable glass flask with a stirrer and the flask inside temperature was started to increase, while stirring the mixture. When the inside temperature reached to 50°C. 50.4g (0.1 mole) of fluoroalcohol $RfC_2H_4OH$ (average molecular weight: 504; Rf: a perfluoroalkyl group having an average carbon atom number of 8.8) dissolved in 41.4g of MMA and 13.8g of EMA was dropwise added to the mixture solution. while monitoring the inside temperature. When the inside temperature was slowly elevated to 70°C due to the exothermic reaction, rate of dropwise addition was adjusted to keep that temperature. Time required from the start to the end of dropwise addition was one hour. After the dropwise addition, the reaction solution was subjected to aging for 3 hours, and then a small amount of the reaction solution was sampled and subjected to infrared spectroscopic analysis. Absorption due to -NCO absorption was observed at 2270 $cm^{-1}$.

10.2g (0.1 mole) of ethyleneglycol monoallyl ether dissolving 0.04g of hydroquinone was slowly dropwise added to the reaction solution so as not to cause vigorous heat generation. Rate of dropwise addition was adjusted to keep the reaction temperature at 70°C. Time for the dropwise addition was one hour. After further aging for one hour, a small amount of the reaction solution was sampled and subjected to infrared spectroscopic analysis and nuclear magnetic resonance determination. It was found that the absorption at 2270 $cm^{-1}$ due to the -NCO absorption disappeared. Amount of the reaction solution was 136.8g with the reaction yield of 99%. The results of nuclear magnetic resonance determination are given below:

$^1$H-NMR ($CD_3COCD_3$) :

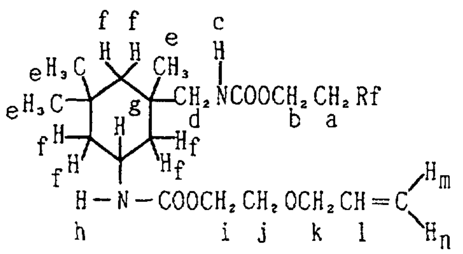

| | Chemical shift(ppm) | Proton number |
|---|---|---|
| a | 2.60 | 2 |
| b | 4.34 | 2 |
| c | 6.17 | 1 |
| h | 6.21 | 1 |
| d | 2.90 | 2 |
| e | 0.98 | 9 |
| f | 0.8~1.79 | 6 |
| g | 3.79 | 1 |
| i | 4.12 | 2 |
| j | 3.58 | 2 |
| k | 3.99 | 2 |
| l | 5.90 | 1 |
| m | 5.12 | 1 |
| n | 5.26 | 1 |

(Total 33)

$^{19}$F-NMR ($CD_3$ $COCD_3$) :

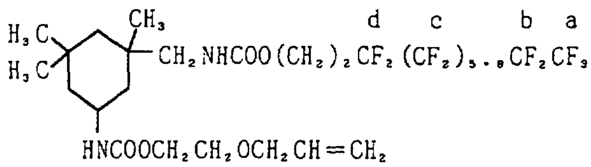

| | Chemical shift($CFCl_3$ ~) | F number |
|---|---|---|
| a | -80.17 | 3 |
| b | -125.21 | 2 |
| c | -120.76 ~ -122.51 | 11.6 |
| d | -112.38 | 2 |
| | | (Total 18.6) |

Whole amount of the reaction product was charged into a separable glass flask, and then 4.6g of polyoxyethylene phenyl ether (Emulgen 950), 0.35g of n-dodecyl mercaptan and 385g of water were added thereto, followed by emulsification by a high speed, rotating homomixter (Ultra-talax T25) at 13000 rpm for 5 minutes.

Thereafter, the flask was flushed with a nitrogen gas for 30 minutes with stirring. After elevation of the inside temperature to 40°C by heating, 1.4g of M-MAM and an aqueous solution containing 2.6g of 2,2'-azobis(2-amidinopropane) dihydrochloride dissolved in 30g of water were added to the reaction system, followed by copolymerization reaction at 70°C for 4 hours, whereby a stable milky-white aqueous emulsion having a solid concentration of 25% by weight was obtained.

COMPARATIVE EXAMPLE 1

In Example 1, 130g of 2-hydroxyethyl methacrylate was used in place of ethyleneglycol monoallyl ether, whereby a solution of fluoroalkylated urethane methacrylate in MIBK having a solid concentration of 40% by weight was obtained. A portion of the MIBK solution was sampled and subjected to solvent removal by distillation. The resulting brownish solid residue was determined for melting point and IR absorption. The results of determination are as follows:

Melting point: 65-91°C
IR absorption: 1700 $cm^{-1}$ (-NHCOO-)
1100-1300 $cm^{-1}$ (-CF)

150g of the MIBK solution (60g as fluoroalkylated urethane methacrylate) was charged into a separable glass flask and subjected to copolymerization reaction in the same manner as in Example 6 except that 180.7g of water was used, whereby a milky-white aqueous emulsion having a solid concentration of 20% by weight was obtained. Scums and precipitates were found on the stirring vanes of the flask.

COMPARATIVE EXAMPLE 2

17.4g (0.1 mole) of 2,4-tolylenediisocyanate and 0.29g of dibutyl tin dilaurate were charged into a separable glass flask with a stirrer, and the flask inside temperature was started to increase, while stirring the mixture. When the inside temperature reached 50°C, 50.4g (0.1 mole) of fluoroalcohol $RfC_2H_4OH$ (average molecular weight: 504; Rf: a perfluoroalkyl group having an average carbon atom number of 8.8) dissolved in 73.2g of MMA and 22g of EMA was dropwise added to the mixture solution, while monitoring the inside temperature. When the inside temperature was slowly elevated to 70° due to the exothermic reaction, rate of dropwise addition was adjusted to keep that temperature. Time required from the start to the end of dropwise addition was one hour. After the dropwise addition, the reaction solution was subjected to aging for 30 minutes, and then a small amount of the reaction solution was sampled and subjected to infrared spectroscopic analysis. Absorption due to -NCO absorption was observed at 2270 $cm^{-1}$.

Then, 75g (0.1 mole) of $CH_2$=$CHCH_2O(C_2H_4O)_{10\ 9}[CH(CH_3)CH_2O]_{38}H$ (Unisafe PKA-5011, trademark of a product commercially available from NOF Corp., Japan) dissolving 0.07g of hydroquinone was slowly dropwise added to the reaction solution so as not to cause vigorous heat generation. Rate of dropwise addition was adjusted to keep

the reaction temperature at 70°C. Time required for the dropwise addition was one hour. Then, the reaction solution was subjected to further aging for one hour, and a small amount of the reaction solution was sampled and subjected to infrared spectroscopic analysis. It was found that the absorption at 2270 $cm^{-1}$ due to the -NCO absorption disappeared. After removal of MMA and EMA from the sample by distillation, the resulting ocherous, viscous materials were determined for IR absorption. The results are given below:

IR absorption: 1700 $cm^{-1}$ (-NHCOO-)
1100-1300 $cm^{-1}$ (-CF)

The reaction mixture was obtained as 235.8g of a monomer mixture solution (recovery yield: 99%), 99g of which was charged into a separable glass flask. Then, 3.3g of polyoxyethylene phenyl ether (Emulgen 950), 0.25g of n-dodecyl mercaptan and 270g of water were added thereto, followed by emulsification by a high speed rotating homomixer (Ultra-talax T25) at 13000 rpm for 5 minutes.

Then, the flask was flushed with a nitrogen gas for 30 minutes with stirring, and after elevation of the inside temperature to 40°C by heating, 1.0g of M-MAM and 2.0g of 2,2'-azobis(2-amidinopropane) dihydrochloride (V-50) dissolved in 30g of water were added to the reaction system, followed by copolymerization reaction at 70°C for 4 hours. With progress of polymerization reaction, precipitates were formed. At the end of polymerization reaction most of the resulting copolymers was settled down as precipitates and no stable aqueous emulsion was obtained.

COMPARATIVE EXAMPLE 3

17.4g (0.1 mole) of 2,4-tolylenediisocyanate and 0.18g of dibutyl tin laurate were charged into a separable glass flask with a stirrer, and the flask inside temperature was started to increase while stirring the mixture. When the inside temperature reached to 50°C, 50.4g (0.1 mole) of fluoroalcohol $RfC_2H_4OH$ (average molecular weight: 504; Rf: a perfluoroalkyl group having an average carbon atom numbver of 8.8) dissolved in 44g 44g of MMA and 14g of EMA was dropwise added to the mixture solution, while monitoring the inside temperature. After slow elevation of the inside temperature to 70°C due to the exothermic reaction, rate of dropwise addition was adjusted to keep that temperature. Time required from the start to the end of dropwise addition was one hour. After the dropwise addition, the reaction solution was subjected to aging for 30 minutes, and then a small amount of the reaction solution was sampled and subjected to infrared spectroscopic analysis. Absorption due to -NCO absorption was observed at 2270 $cm^{-1}$.

20g (0.1 mole) of $CH_2=CHCH_2O(C_2H_4O)_{3.2}H$ (Uniox PKA-5001, trademark of a product commercially available from NOF Corp., Japan) dissolving 0.04g of hydroquinone was slowly dropwise added to the reaction solution so as not to cause vigorous heat generation. Rate of dropwise addition was adjusted to keep the reaction temperature at 70°C. Time required for the dropwise addition was one hour. The reaction solution was subjected to further aging for one hour, and a small amount of the reaction solution was sampled and subjected to infrared spectroscopic analysis. It was found that the absorption at 2270 $cm^{-1}$ due to the -NCO absorption disappeared. After removal of MMA and EMA from the sample by distillation, the resulting ocherous viscous materials were determined for IR absorption. The results are as follows:

IR absorption: 1700 $cm^{-1}$ (-NHCOO-)
1100-1300 $cm^{-1}$ (-CF)

The reaction solution was obtained as 144.5g of a monomer mixture solution (recovery yield: 99%), 99g of which was subjected to copolymerization reaction in the same manner as in Comparative Example 2, whereby a milky-white aqueous emulsion having a solid concentration of 25% by weight was obtained.

COMPARATIVE EXAMPLE 4

Copolymerization reaction was carried out in the same manner as in Comparative Example 2, using 60g of fluoroalkyl acrylate $RfC_2H_4OCOCH=CH_2$ (Rf: a perfluoroalkyl group having an average carbon atom number of 8.8), 30g of MMA and 9g of EMA, whereby a milky-white aqueous emulsion having a solid concentration of 25% by weight was obtained.

COMPARATIVE EXAMPLE 5

17.4g (0.1 mole) of 2,4-tolylenediisocyanate and 0.15g of dibutyl tin dilaurate were charged into a separable glass flask with a stirrer, and the flask inside temperature was started to increase, while stirring the mixture. When the inside temperature reached to 50°C, 50.4g (0.1 mole) of fluoroalcohol $RfC_2H_4OH$ (average molecular weight: 504; Rf:

a perfluoroalkyl group having an average carbon atom number of 8.8) dissolved in 37.8g of MMA and 11.3g of EMA was dropwise added to the mixture solution, while monitoring the inside temperature. After slow elevation of the inside temperature to 70°C due to the exothermic reaction, rate of dropwise addition was adjusted to keep that temperature. Time required from the start to the end of dropwise addition was one hour. After the dropwise addition, the reaction solution was subjected to aging for 30 minutes and a small amount of the reaction solution was sampled and subjected to infrared spectroscopic analysis. Absorption due to -NCO absorption was observed at 2270 $cm^{-1}$.

5.8g (0.1 mole) of allyl alcohol dissolving 0.04g of hydroquinone was slowly dropwise added to the reaction solution so as not to cause vigorous heat generation. Rate of dropwise addition was adjusted to keep the reaction temperature at 70°C. Time required for the dropwise addition was one hour. The reaction solution was subjected to further aging for one hour, and a small amount of the reaction solution was sampled and subjected to infrared spectroscopic analysis. It was found that the absorption at 2270 $cm^{-1}$ due to the -NCO absorption disappeared. After removal of MMA and EMA from the sample by distillation, the resulting brownish solids were determined for melting point and IR absorption. The results are given below:

| | |
|---|---|
| Melting point: | 90-96°C |
| IR absorption: | 1700 $cm^{-1}$ (-NHCOO-) |
| | 1100-1300 $cm^{-1}$ (-CF) |

The reaction solution was obtained as 122.9g of a monomer mixture solution (recovery yield: 99%), 99g of which was subjected to copolymerization reaction in the same manner as in Comparative Example 2, whereby a milky-white, aqueous emulsion having a solid concentration of 21% by weight was obtained. With progress of polymerization reaction, precipitates were started to form.

The aqueous emulsions obtained in the foregoing Examples and Comparative Examples were each diluted with water to a solid concentration of 0.5% by weight and sprayed onto polyamide carpets of undyed cut piles by an atomizer to a wet pickup (ratio of the deposited emulsion to the weight of carpet) of 65%. Then, the wet carpets were placed in a hot air-circulating drier and dried at 130°C for 30 minutes.

The carpets so treated were tested for the following test items:

Water-repellency: Maximum % by weight of isopropanol (IPA) capable of retaining droplets of mixture solutions of isopropanol and water on the carpets at 30 seconds after trickling, when the mixture solutions trickled thereon. Evaluation of water-repellency is made in 11 rankings between 0 and 100, so that evaluation with e.g. an aqueous 30 wt.% IPA solution can have a ranking of 30. Ranking of 0 means complete permeation of water. Water-repellency increases with a higher ranking. Ranking of 100 means no permeation even with 100% IPA.

Oil-repellency according to AATCC TM 118-1992:

Test solutions having various surface tensions [0: none; 1: Nujol; 2: Nujol-n-hexadecane (65:35) mixture solution; 3: n-hexadecane; 4: n-tetradecane; 5: n-dodecane; 6: n-decane; 7: n-octane; 8: n-heptane] are trickled onto the carpets and evaluation of oil-repellency is made as a maximum value at which droplets of test solutions undergo no permeation even 30 seconds after the trickling. Oil-repellency of 0 means that no droplets of Nujol can be retained at all at 30 seconds after the trickling, that is, the carpets have no oil resistance at all, and oil-repellency of 8 means the highest oil-repellency, for example, droplets of n-heptane can be retained even 30 seconds after the trickling.

Anti-dry soiling property according to JIS L-1023-1992:

A circular carpet is fixed onto a disc plate on a circular tester and rotated, and a standard soiling material in a composition comprising 40% by weight of peat moss, 17% by weight of portland cement, 17% by weight of kaolin, 17% by weight of diatomaceous earth, 0.1% by weight of carbon black, 0. 15% by weight of iron oxide (III) for ferrite and 8.75% by weight of Nujol is sprayed thereon through two sets of impact rotors, while tapping to soil the carpet. Then, excess standard soiling material is removed therefrom by suction with a vacuum cleaner and lightness is measured by a colorimeter to make evaluation of the anti-dry soiling property.

More specifically, 20g of the standard soiling material is filled into the two sets of impact rotors, and to make uniform spraying of the standard soiling material onto the test piece before the main test, a preliminary test piece is fixed onto the disc plate and 100 trial tappings are carried out. Thereafter, the main test is carried out. In the main test, a test piece is fixed on the disc plate and soiled, while making 50 revolutions of the disc plate. Immediately after the soiling, the test piece is vacuum cleaned while making 20 revolutions of the disc plate. Then, one more soiling is carried out, while making 50 revolutions of the disc plate. Immediately after the soiling, the test piece is vacuum cleaned. The foregoing operations are carried out two test pieces.

Evaluation of anti-dry soiling property is made by measuring a presoiling lightness $L0^*$ and a postsoiling lightness $L1^*$ each of the two test pieces by a colorimeter (CM-2002, trademark of a meter commercially available from Minolta Camera Co., Ltd, Japan), followed by calculation according to the following calculation equation. An evaluation value is an average degree of soiling of the two carpet test pieces. As the color order system, an $L^*a^*b^*$ color order system set forth by JIS Z-8729-1980 is used.

$$\text{Degree of soiling} = \frac{L0^* - L1^*}{L0^*} \times 100$$

Emulsion stability: At the end of copolymerization reaction, complete absence of precipitates are marked as O; presence of a small amount of precipitates as Δ; and presence of a very large amount of precipitates as ×.

Test results are given in the following Table.

| Example | Water-repellency | Oil-repellency | Degree of soiling | Stability |
|---|---|---|---|---|
| (Untreated) | 0 | 0 | 28.9 | - |
| Example 2 | 30 | 3 | 11.3 | ○ |
| " 3 | " | 3 | 10.4 | ○ |
| " 4 | 40 | 4 | 9.6 | ○ |
| " 5 | " | " | 9.4 | ○ |
| " 6 | " | " | 8.3 | ○ |
| " 7 | " | " | 8.4 | ○ |
| " 8 | " | " | 7.9 | ○ |
| " 9 | " | " | 10.3 | ○ |
| " 10 | " | " | 10.1 | ○ |
| " 11 | " | " | 6.8 | ○ |
| " 12 | " | " | 8.7 | ○ |
| " 13 | " | " | 9.1 | ○ |
| Comp. Ex. 1 | 30 | 3 | 20.5 | Δ |
| " 3 | " | " | 19.8 | ○ |
| " 4 | " | 2 | 20.3 | Δ |
| " 5 | " | 3 | 17.5 | × |

The following is observations of results of Comparative

Examples:

(1) In Comparative Example 1 as a trace test of JP-B-3-3542, precipitates were formed during the copolymerization reaction, and the water-repellency and the oil-repellency were poorer each by one ranking and the degree of soiling was poorer by 13.7 points than those of Example 11 using reaction product of 2,4-tolylenediisocyate with fluoroalcohol $RfC_2H_4OH$, followed by reaction with ethyleneglycol monoallyl ether.

(2) In Comparative Example 3 using reaction product of 2,4-tolylenediisocyanate with fluoroalcohol $RfC_2H_4OH$, followed by reaction with $CH_2=CHCH_2O(C_2H_4O)_nH$, a stable emulsion could be obtained, but the water-repellency and the oil-repellency were poorer each by one ranking and the degree of soiling was poorer by 13 points than those of Example 11.

(3) In Comparative Example 4 using a fluoroalkyl acrylate as a good water-repellent, precipitates were formed during the copolymerization, and the water-repellency was poorer by one ranking, the oil-repellency by two rankings and the degree of soiling by 13.5 points than those of Example 11.

(4) In Comparative Example 5 using reaction product of 2,4-tolylenediisocyanate with fluoroalcohol $RfC_2H_4OH$, followed by reaction with allyl alcohol, a large amount of precipitates was formed during the copolymerization, and the water-repellency and the oil-repellency were poorer each by one ranking and the degree of soiling was poorer by 10.7 points than those of Example 11.

## Claims

1. A fluoroalkylated allylurethane represented by the following general formula:

$$RfROCONHR'\ NHCOOC_nH_{2n}\ OCH_2\ CH{=}CH_2$$

where Rf is a perfluoroalkyl group having 2 to 20 carbon atoms, R is a divalent organic group, R' is a diisocyanate residue and n is an integer of 2 to 6.

**2.** A process for preparing a fluoroalkylated allylurethane represented by the following general formula:

$$RfROCONHR'\ NHCOOC_nH_{2n}\ OCH_2\ CH{=}CH_2$$

where Rf is a perfluoroalkyl group having 2 to 20 carbon atoms, R is a divalent organic group, R' is a diisocyanate residue and n is an integer of 2 to 6, which comprises reacting a diisocyanate compound having two NCO groups of different reactivity in the molecule with a fluoroalcohol represented by the following general formula:

RfROH

where Rf and R have the same meanings as defined above, followed by reaction with an alkyleneglycol monoallyl ether represented by the following general formula:

$$HOC_n\ H_{2n}\ OCH_2\ CH{=}CH_2$$

where n has the same meaning as defined above.

**3.** A process according to Claim 2, wherein the diisocyanate compound having two NCO groups of different reactivity in the molecule is 2,4-tolylenediisocyanate,

**4.** A process according to Claim 2, wherein the diisocyanate compound having two NCO groups of different reactivity is 3-isocyanatomethyl-3,5,5-trimethylcyclohexyl isocyanate.

**5.** A process according to Claim 2, wherein the reaction is carried out in an inert organic solvent.

**6.** A process according to Claim 5, wherein the inert organic solvent is a ketone, an ester or an aromatic hydrocarbon.

**7.** A process according to Claim 2, wherein the reaction is carried out in a polymerizable monomer.

**8.** A process according to Claim 7, wherein the polymerizable monomer is a methacrylate ester, an acrylate ester or vinylidene chloride.

**9.** A process for preparing a fluoroalkylated allylurethane represented by the following general formula:

$$RfROCONHR'NHCOOC_nH_{2n}OCH_2CH{=}CH_2$$

where Rf is a perfluoroalkyl group having 2 to 20 carbon atoms, R is a divalent organic group, R' is a diisocyanate residue and n is an integer of 2 to 6, which comprises reacting a diisocyanate compound having two NCO groups of different reactivity in the molecule with an alkyleneglycol monoallyl ether represented by the following general formula:

$$HOC_nH_{2n}OCH_2CH{=}CH_2$$

where n has the same meaning as defined above, followed by reaction with a fluoroalcohol represented by the following general formula:

$$RfROH$$

where Rf and R have the same meanings as defined above.

**10.** A process according to Claim 9, wherein the diisocyanate compound having two NCO groups of different reactivity in the molecule is 2,4-tolylenediisocyanate.

**11.** A process according to Claim 9, wherein the diisocyanate compound having two NCO groups of different reactivity is 3-isocyanatomethyl-3,5,5-trimethylcyclohexyl isocyanate.

**12.** A process according to Claim 9, wherein the reaction is carried out in an inert organic solvent.

**13.** A process according to Claim 12, wherein the organic solvent is a ketone, an ester or an aromatic hydrocarbon.

**14.** A process according to Claim 9, wherein the reaction is carried out in a polymerizable monomer.

**15.** A process according to Claim 14, wherein the polymerizable monomer is a methacrylate ester, an acrylate ester or vinylidene chloride.

**16.** A copolymer obtained by copolymerization of a fluoroalkylated allylurethane represented by the following general formula:

$$RfROCONHR'NHCOOC_nH_{2n}OCH_2CH=CH_2$$

where Rf is a perfluoroalkyl group having 2 to 20 carbon atoms, R is a divalent organic group, R' is a diisocyanate residue and n is an integer of 2 to 6, with at least one of a methacrylate ester, an acrylate ester and vinylidene chloride.

**17.** A copolymer according to Claim 16, wherein the copolymer is obtained by copolymerizing about 30 to about 90% by weight of the fluoroalkylated allylurethane on the basis of total comonomers.

**18.** A process for preparing an aqueous copolymer emulsion which comprises copolymerizing a fluoroalkylated allylurethane represented by the following general formula:

$$RfROCONHR'\ NHCOOC_nH_{2n}OCH_2CH=CH_2$$

where Rf is a perfluoroalkyl group having 2 to 20 carbon atoms, R is a divalent organic group, R' is a diisocyanate residue and n is an integer of 2 to 6, with at least one of a methacrylate ester, an acrylate ester and vinylidene chloride by emulsion polymerization.

**19.** A water- and oil-repellent, anti-soiling processing agent, which comprises a copolymer obtained by copolymerization of a fluoroalkylated allylurethane represented by the following general formula:

$$RfROCONHR'NHCOOC_nH_{2n}OCH_2CH=CH_2$$

where Rf is a perfluoroalkyl group having 2 to 20 carbon atoms, R is a divalent organic group, R' is a diisocyanate residue and n is an integer of 2 to 6, with at least one of a methacrylate ester, an acrylate ester and vinylidene chloride as an effective component

**20.** A water- and oil-repellent, anti-soiling processing agent according to Claim 19, which is used in an aqueous emulsion.

**Patentansprüche**

1. Fluoralkyliertes Allylurethan, dargestellt durch die folgende allgemeine Formel:

$$RfROCONHR'NHCOOC_nH_{2n}OCH_2CH=CH_2$$

worin Rf eine Perfluoralkyl-Gruppe mit 2 bis 20 Kohlenstoffatomen darstellt, R bedeutet eine divalente organische Gruppe, R' bedeutet einen Diisocyanat-Rest und n ist eine ganze Zahl von 2 bis 6.

2. Verfahren zur Herstellung eines fluoralkylierten Allylurethans, dargestellt durch die folgende allgemeine Formel:

$$RfROCONHR'NHCOOC_nH_{2n}OCH_2CH=CH_2$$

worin Rf eine Perfluoralkyl-Gruppe mit 2 bis 20 Kohlenstoffatomen bedeutet, R ist eine divalente organische Gruppe, R' ist ein Diisocyanat-Rest und n ist eine ganze Zahl von 2 bis 6, das die Umsetzung einer Diisocyanat-Verbindung mit zwei NCO-Gruppen unterschiedlicher Reaktivität in dem Molekül mit einem Fluoralkohol umfaßt, dargestellt durch die folgende allgemeine Formel:

$$RfROH$$

worin Rf und R dieselben Bedeutungen haben wie oben definiert, gefolgt von einer Umsetzung mit einem Alkylenglycolmonoallylether, dargestellt durch die folgende allgemeine Formel :

$$HOC_nH_{2n}OCH_2CH=CH_2$$

worin n dieselbe Bedeutung hat wie oben definiert.

3. Verfahren gemäß Anspruch 2, wobei die Diisocyanat-Verbindung mit zwei NCO-Gruppen unterschiedlicher Reaktivität im Molekül 2,4-Tolylendiisocyanat ist.

4. Verfahren gemäß Anspruch 2, wobei die Diisocyanat-Verbindung mit zwei NCO-Gruppen unterschiedlicher Reaktivität 3-Isocyanatomethyl-3,5,5-trimethylcyclohexylisocyanat ist.

5. Verfahren gemäß Anspruch 2, wobei die Reaktion in einem inerten organischen Lösungsmittel durchgeführt wird.

6. Verfahren genäß Anspruch 5, wobei das inerte organische Lösungsmittel ein Keton, ein Ester oder ein aromatischer Kohlenwasserstoff ist.

7. Verfahren gemäß Anspruch 2, wobei die Reaktion in einem polymerisierbaren Monomer durchgeführt wird.

8. Verfahren gemäß Anspruch 7, wobei das polymerisierbare Monomer ein Methacrylatester, ein Acrylatester oder Vinylidenchlorid ist.

9. Verfahren zur Herstellung eines fluoralkylierten Allylurethans, dargestellt durch die folgende allgemeine Formel:

$$RfROCONHR'NHCOOC_nH_{2n}OCH_2CH=CH_2$$

worin Rf eine Perfluoralkyl-Gruppe mit 2 bis 20 Kohlenstoffatomen ist, R ist eine divalente organische Gruppe, R' ist ein Diisocyanat-Rest und n ist eine ganze Zahl von 2 bis 6, das die Umsetzung einer Diisocyanat-Verbindung mit zwei NCO-Gruppen unterschiedlicher Reaktivität im Molekül mit einem Alkylenglycolmonoallylether umfaßt, dargestellt durch die folgende allgemeine Formel:

$$HOC_nH_{2n}OCH_2CH=CH_2$$

worin n dieselbe Bedeutung wie oben definiert aufweist, gefolgt durch eine Umsetzung mit einem Fluoralkohol, dargestellt durch die folgende allgemeine Formel:

$$RfROH$$

worin Rf und R dieselbe Bedeutung wie oben definiert haben.

**10.** Verfahren gemäß Anspruch 9, wobei die Diisocyanat-Verbindung mit zwei NCO-Gruppen unterschiedlicher Reaktivität im Molekül 2,4-Tolylendiisocyanat ist.

**11.** Verfahren gemäß Anspruch 9, worin die Diisocyanat-Verbindung mit zwei NCO-Gruppen unterschiedlicher Reaktivität 3-Isocyanatomethyl-3,5,5-trimethylcyclohexylisocyanat ist.

**12.** Verfahren gemäß Anspruch 9, wobei die Reaktion in einem inerten organischen Lösungsmittel durchgeführt wird.

**13.** Verfahren gemäß Anspruch 12, wobei das organische Lösungsmittel ein Keton, ein Ester oder eine aromatischer Kohlenwasserstoff ist.

**14.** Verfahren gemäß Anspruch 9, wobei die Reaktion in einem polymerisierbaren Monomer durchgeführt wird.

**15.** Verfahren gemäß Anspruch 14, wobei das polymerisierbare Monomer ein Methacrylatester, ein Acrylatester oder Vinylidenchlorid ist.

**16.** Copolymer, erhalten durch Copolymerisation eines fluoralkylierten Allylurethans, dargestellt durch die folgende allgemeine Formel:

$$RfROCONHR'NHCOOC_nH_{2n}OCH_2CH=CH_2$$

worin Rf eine Perfluoralkyl-Gruppe mit 2 bis 20 Kohlenstoffatomen ist, R ist eine divalente organische Gruppe, R' ist ein Diisocyanat-Rest und n ist eine ganze Zahl von 2 bis 6, mit mindestens einem Methacrylatester, einem Acrylatester und Vinylidenchlorid.

**17.** Copolymer gemäß Anspruch 16, wobei das Copolymer durch Copolymerisation von ungefähr 30 bis ungefähr 90 Gew.-% des fluoralkylierten Allylurethans auf der Basis der gesamten Comonomere erhalten wird.

**18.** Verfahren zur Herstellung einer wäßrigen Copolymer-Emulsion, das die Copolymerisation eines fluoralkylierten Allylurethans, dargestellt durch die folgende allgemeine Formel:

$$RfROCONHR'NHCOOC_nH_{2n}OCH_2CH=CH_2$$

worin Rf eine Perfluoralkyl-Gruppe mit 2 bis 20 Kohlenstoffatomen ist, R ist eine divalente organische Gruppe, R' ist ein Diisocyanat-Rest und n ist eine ganze Zahl von 2 bis 6 mit mindestens einem Methacrylatester, einem Acrylatester oder einem Vinylidenchlorid durch Emulsionspolymerisation umfaßt.

**19.** Ein Wasser- und Öl-abweisendes, gegen eine Verschmutzung wirkendes Verarbeitungsmittel, das ein Copolymer umfaßt, erhalten durch Copolymerisation eines fluoralkylierten Allylurethans, dargestellt durch die folgende allgemeine Formel:

$$RfROCONHR'NHCOOC_nH_{2n}OCH_2CH=CH_2$$

worin Rf eine Perfluoralkyl-Gruppe mit 2 bis 20 Kohlenstoffatomen ist, R ist eine divalente organische Gruppe, R'

ist ein Diisocyanat-Rest und n ist eine ganze Zahl von 2 bis 6, mit mindestens einem Methacrylatester, einem Acrylatester und Vinylidenchlorid als effektiven Bestandteil.

**20.** Wasser- und Öl-abweisendes, einer Verschmutzung entgegenwirkendes Verarbeitungsmittel gemäß Anspruch 19, das in einer wäßrigen Emulsion verwendet wird.

**Revendications**

**1.** Un allyluréthane fluoroalkylé représenté par la formule générale suivante :

$$RfROCONHR'NHCOOC_nH_{2n}OCH_2CH = CH_2$$

dans laquelle Rf est un groupe perfluoroalkyle ayant de 2 à 20 atomes de carbone, R est un groupe organique divalent, R' est un résidu diisocyanate et n est un nombre entier de 2 à 6.

**2.** Un procédé pour préparer un allyluréthane fluoroalkylé représenté par la formule générale suivante :

$$RfROCONHR'NHCOOC_nH_{2n}OCH_2CH = CH_2$$

dans laquelle Rf est un groupe perfluoroalkyle ayant de 2 à 20 atomes de carbone, R est un groupe organique divalent, R' est un résidu diisocyanate et n est un nombre entier de 2 à 6, qui comprend la réaction d'un composé diisocyanate ayant deux groupes NCO de réactivité différente dans la molécule avec un alcool fluoré représenté par la formule générale suivante :

$$RfROH$$

dans laquelle Rf et R ont les mêmes significations que celles définies ci-dessus, suivie par la réaction avec un ether monoallylique d'alkylène glycol représenté par la formule générale suivante :

$$HOC_nH_{2n}OCH_2CH = CH_2$$

dans laquelle n a la même signification que celle définie ci-dessus.

**3.** Un procédé selon la revendication 2, dans lequel le composé diisocyanate ayant deux groupes NCO de réactivité différente dans la molécule est le 2,4-tolyléne diisocyanate.

**4.** Un procédé selon la revendication 2, dans lequel le composé diisocyanate ayant deux groupes NCO de réactivité différente est le 3-isocyanatométhyl-3,5,5-triméthylcyclohexyl isocyanate.

**5.** Un procédé selon la revendication 2, dans lequel la réaction est effectuée dans un solvant organique inerte.

**6.** Un procédé selon la revendication 5, dans lequel le solvant organique inerte est une cétone, un ester ou un hydrocarbure aromatique.

**7.** Un procédé selon la revendication 2, dans lequel la réaction est effectuée dans un monomère polymérisable.

**8.** Un procédé selon la revendication 7, dans lequel le monomère polymérisable est un ester de méthacrylate, un ester d'acrylate ou le chlorure de vinylidène.

**9.** Un procédé pour préparer un allyluréthane fluoroalkylé représenté par la formule générale suivante :

$$RfROCONHR'NHCOOC_nH_{2n}OCH_2CH = CH_2$$

dans laquelle Rf est un groupe perfluoroalkyle ayant de 2 à 20 atomes de carbone, R est un groupe organique divalent, R' est un résidu diisocyanate et n est un nombre entier de 2 à 6, qui comprend la réaction d'un composé diisocyanate ayant deux groupes NCO de réactivité différente dans la molécule avec un ether monoallylique d'alkylène glycol représenté par la formule générale suivante:

$$HOC_nH_{2n}OCH_2CH = CH_2$$

dans laquelle n a la même signification que celle définie ci-dessus, suivie par la réaction avec un alcool fluoré représenté par la formule générale suivante:

$$RfROH$$

dans laquelle Rf et R ont les mêmes significations que celles définies ci-dessus.

**10.** Un procédé selon la revendication 9, dans lequel le composé diisocyanate ayant deux groupes NCO de réactivité différente dans la molécule est le 2,4-tolylène diisocyanate.

**11.** Un procédé selon la revendication 9, dans lequel le composé diisocyanate ayant deux groupes NCO de réactivité différente dans la molécule est le 3-isocyanatométhyl-3,5,5-triméthylcyclohexyl isocyanate.

**12.** Un procédé selon la revendication 9, dans lequel la réaction est effectuée dans un solvant organique inerte.

**13.** Un procédé selon la revendication 12, dans lequel le solvant organique est une cétone, un ester ou un hydrocarbure aromatique.

**14.** Un procédé selon la revendication 9, dans lequel la réaction est effectuée dans un monomére polymérisable.

**15.** Un procédé selon la revendication 14, dans lequel le monomère polymérisable est un ester de méthacrylate, un ester d'acrylate ou le chlorure de vinylidène.

**16.** Un copolymère obtenu par la copolymérisation d'un allyluréthane fluoroalkylé représenté par la formule générale suivante :

$$RfROCONHR'NHCOOC_nH_{2n}OCH_2CH = CH_2$$

dans laquelle Rf est un groupe perfluoroalkyle ayant de 2 à 20 atomes de carbone, R est un groupe organique divalent, R' est un résidu diisocyanate et n est un nombre entier de 2 à 6, avec au moins l'un d' un ester de méthacrylate, un ester d'acrylate et du chlorure de vinylidène.

**17.** Un copolymère selon la revendication 16, dans lequel le copolymère est obtenu en copolymérisant environ 30 à environ 90% en poids de l' allyluréthane fluoroalkylé sur la base de la totalité des comonomères.

**18.** Un procédé pour préparer une émulsion aqueuse de copolymère, qui comprend la copolymérisation d'un allyluréthane fluoroalkylé représenté par la formule générale suivante :

$$RfROCONHR'NHCOOC_nH_{2n}OCH_2CH = CH_2$$

dans laquelle Rf est un groupe perfluoroalkyle ayant de 2 à 20 atomes de carbone, R est un groupe organique divalent, R' est un résidu diisocyanate et n est un nombre entier de 2 à 6, avec au moins l'un d' un ester de méthacrylate, un ester d'acrylate et du chlorure de vinylidène par polymérisation en émulsion.

**19.** Un agent de traitement anti-taches hydrofuge et lipofuge, qui comprend un copolymère obtenu par la copolymérisation d'un allyluréthane fluoroalkylé représenté par la formule générale suivante :

$$RfROCONHR'NHCOOC_nH_{2n}OCH_2CH = CH_2$$

dans laquelle Rf est un groupe perfluoroalkyle ayant de 2 à 20 atomes de carbone, R est un groupe organique divalent, R' est un résidu diisocyanate et n est un nombre entier de 2 à 6, avec au moins l'un d' un ester de méthacrylate, un ester d'acrylate et du chlorure de vinylidène, en tant que composé efficace.

**20.** Un agent de traitement anti-taches hydrofuge et lipofuge selon la revendication 19, qui est utilisé dans une émulsion aqueuse.